# EUROPEAN PATENT APPLICATION

(11) **EP 4 830 833 A1**
(43) Date of publication of application: **28.10.2026**
(21) Application number: 26162974.5
(22) Date of filing: 06.03.2026
(51) Int. Cl.: B01J 21/06, B01J 23/00, B01J 23/83, B01J 35/61, B01J 35/64, B01J 37/02, B01J 37/08, B01J 37/18, C07C 51/00, B01J 23/755, B01J 35/63

(54) **CATALYST, PREPARATION METHOD AND USE THEREOF**

(30) Priority: 07.03.2025 CN 202510267601
(71) Applicant: Shanghai DiYang Chemical Technology Co., Ltd., Shanghai 200135 (CN)
(72) Inventor: ZHANG, Qiuming, Shanghai, 200135 (CN); CHENG, Ru, Shanghai, 200135 (CN); MA, Qiang, Shanghai, 200135 (CN); WANG, Xudong, Shanghai, 200135 (CN); SHI, Zhijie, Shanghai, 200135 (CN)
(74) Representative: Nederlandsch Octrooibureau

(57) **Abstract**

The present disclosure belongs to the fields of catalysts and petrochemical engineering, and specifically relates to a catalyst, as well as its preparation method and use thereof. The catalyst comprises a composite carrier and an active metal Ni loaded on the composite carrier; the composite carrier is formed by compounding a modifier and a natural mineral, wherein the modifier comprises zirconium and a rare earth metal; the natural mineral is one or more selected from diatomite, sepiolite, halloysite, attapulgite, vermiculite and molybdenite; the rare earth metal is one or more selected from yttrium, lanthanum, cerium, neodymium, samarium, europium, gadolinium and ytterbium. The catalyst of the present disclosure exhibits excellent structural stability, as well as high activity and high selectivity in the catalytic preparation of DMS. Particularly, the catalyst of the present disclosure exhibits excellent performance in the hydrogenation of DMM to produce DMS, which can effectively improve the selectivity to DMS and suppress the formation of GBL, BDO and THF.

## Description

### Technical Field

The present disclosure belongs to the fields of catalysts and petrochemical engineering, and specifically relates to a catalyst, preparation method and use thereof.

### Background

At present, the main production method of polybutylene succinate (PBS) is the direct esterification polymerization of succinic acid or succinic anhydride with 1,4-butanediol (BDO). However, the PBS synthesized by this method has a relatively low molecular weight, and its color number, mechanical properties and processing properties are difficult to meet the expected requirements, which limits the promotion and use of PBS. According to the report in Polyester Industry (Vol. 36, Issue 2), PBS produced by the transesterification polymerization of dimethyl succinate (DMS) with BDO is more likely to achieve high molecular weight products, and its physical property indicators such as terminal carboxyl group, melt flow index and oxidation resistance are more in line with the requirements of downstream products. Therefore, the development of a low-cost and high-quality polymerization-grade DMS production process has broad use prospects and economic benefits.

Traditional DMS production processes mainly comprise succinic acid esterification, catalytic hydrogenation, and electrochemical catalytic conversion. Due to the use of highly corrosive catalysts such as concentrated sulfuric acid, acidic resins or p-toluenesulfonic acid in succinic acid esterification and electrochemical catalytic methods, coupled with slow reaction rates and numerous by-products, these processes lack the foundation for large-scale industrial plant development and have mostly been eliminated. Currently, the catalytic hydrogenation method is the most promising for industrial use. This process usually uses maleic anhydride as the raw material: first, dimethyl maleate (DMM) is synthesized through esterification, then DMM is converted to DMS via hydrogenation, and finally DMS is obtained through light component removal and heavy component removal. The key of this process lies in the hydrogenation reaction after esterification. Since DMS, BDO, tetrahydrofuran (THF) and γ-butyrolactone (GBL) can be mutually converted through hydrogenation, hydrogenolysis or dehydration reactions, and DMS is usually an intermediate product that is prone to rapid conversion into BDO, GBL or THF, it is difficult to achieve a high yield of DMS, making it unsuitable for separation. Although many literatures have reported methods for producing various chemicals from maleic anhydride through esterification and hydrogenation, most of the products are mixtures of BDO, DMS, THF and GBL. Patent Application CN104822650A discloses a method for obtaining DMS from maleic anhydride and alkanol under the action of a Pd catalyst, but the DMS content in the product is relatively low (only 30%-55%), leading to great difficulties in rectification and separation. Patent Application CN102070448A discloses a method for preparing DMS from maleic anhydride through two steps of esterification and hydrogenation under the action of a noble metal catalyst. Only a few literatures report obtaining DMS through DMM hydrogenation. Patent Applications such as CN117380185A and CN103657693A mainly use noble metal catalysts such as Pd, Pt or Ru to prepare DMS via DMM hydrogenation. However, these methods not only have high costs but also low conversion rates and numerous side reactions, making it difficult to purify the produced DMS to meet the polymerization-grade requirements.

### Summary

An object of the present disclosure is to provide a catalyst, preparation method and use thereof.

A first aspect of the present disclosure provides a catalyst, comprising a composite carrier and an active metal Ni loaded on the composite carrier, wherein the composite carrier is formed by compounding a modifier and a natural mineral, the modifier comprises zirconium and a rare earth metal; the natural mineral is one or more selected from diatomite, sepiolite, halloysite, attapulgite, vermiculite and molybdenite; and the rare earth metal is one or more selected from yttrium, lanthanum, cerium, neodymium, samarium, europium, gadolinium and ytterbium.

In one or more embodiments, the modifier consists of zirconium and the rare earth metal.

In one or more embodiments, a molar ratio of zirconium to the rare earth metal in the modifier is 1:(0.001-0.5).

In one or more embodiments, a content of zirconium element is 0.01-30wt% based on the weight of the natural mineral.

In one or more embodiments, a specific surface area of the composite carrier is 150-450 m²/g.

In one or more embodiments, a total pore volume of the composite carrier is 0.15-1.5 cm³/g.

In one or more embodiments, an average pore diameter of the composite carrier is 5-30 nm.

In one or more embodiments, a content of the active metal Ni in the catalyst is 1-40wt%.

In a second aspect of the present disclosure, a method for preparing the catalyst according to the first aspect of the present disclosure is provided, which comprises the following steps:
(1) preparing a modifier from an aqueous solution containing a zirconium salt and a rare earth metal salt by a high-pressure hydrothermal method;
(2) forming a precipitate from the modifier and the natural mineral in a dispersant, filtering and collecting the precipitate, drying and calcining the precipitate to obtain a composite carrier;
(3) impregnating the composite carrier in an aqueous solution containing a nickel salt, filtering off the solution, drying and calcining to obtain a catalyst precursor;
(4) subjecting the catalyst precursor to a reduction reaction in a reducing gas to obtain the catalyst.

In one or more embodiments, in step (1), the zirconium salt is one or more selected from zirconium nitrate, zirconium chloride and zirconium alkoxide.

In one or more embodiments, in step (1), the rare earth metal salt is one or more selected from Gd(NO₃)₃·6H₂O, Yb(NO₃)₃·5H₂O, Y(NO₃)₃·4H₂O, La(NO₃)₃·6H₂O, Ce(NO₃)₃·6H₂O, Nd(NO₃)₃·6H₂O, Sm(NO₃)₃·6H₂O, Eu(NO₃)₃·5H₂O and chlorides of the rare earth metals.

In one or more embodiments, in step (1), a concentration of the zirconium salt in the aqueous solution is 2-2.5 mol/L.

In one or more embodiments, in step (1), a molar ratio of zirconium to the rare earth metal in the aqueous solution is 1:(0.001-0.5).

In one or more embodiments, in step (1), a reaction pressure of the high-pressure hydrothermal method is 1.0-3.0 MPa.

In one or more embodiments, in step (1), a reaction temperature of the high-pressure hydrothermal method is 110-150 °C.

In one or more embodiments, in step (1), a reaction time of the high-pressure hydrothermal method is 3-24 h.

In one or more embodiments, in step (2), the modifier is added in an amount such that the content of zirconium element in the modifier is 0.01-30wt% based on the weight of the natural mineral.

In one or more embodiments, in step (2), the conditions for forming the precipitate are: dispersing the modifier and the natural mineral under stirring with heating, followed by standing under heating until a precipitate is formed.

In one or more embodiments, in step (2), a drying temperature is 100-150 °C.

In one or more embodiments, in step (2), a drying time is 2-48 h.

In one or more embodiments, in step (2), a calcination temperature is 150-800 °C.

In one or more embodiments, in step (2), a calcination time is 0.5-12 h.

In one or more embodiments, prior to performing step (2), the natural mineral is subjected to heat treatment and/or chemical treatment.

In one or more embodiments, in step (3), the nickel salt is one or more selected from nickel nitrate, nickel chloride and nickel sulfate.

In one or more embodiments, in step (3), the aqueous solution containing the nickel salt further comprises an organic acid.

In one or more embodiments, in step (3), a concentration of the nickel salt in the aqueous solution containing the nickel salt is 0.01-2.0 g/mL.

In one or more embodiments, in step (3), an impregnation time is 20-240 min.

In one or more embodiments, in step (3), a drying temperature is 80-120 °C.

In one or more embodiments, in step (3), a drying time is 2-12 h.

In one or more embodiments, in step (3), a calcination temperature is 150-550 °C.

In one or more embodiments, in step (3), a calcination time is 0.5-50 h.

In one or more embodiments, in step (4), the reducing gas comprises hydrogen and a protective gas, wherein the protective gas is one or more selected from nitrogen, helium and argon.

In one or more embodiments, in step (4), a temperature of the reduction reaction is 130-650 °C.

In one or more embodiments, in step (4), a system for the reduction reaction is heated to a set temperature at a heating rate of not more than 10 °C/h;
In one or more embodiments, in step (4), after the temperature of the system for the reduction reaction reaches the set value, the system is maintained for 4-48 h.

In a third aspect of the present disclosure, a method for preparing dimethyl succinate is provided, which comprises step (a): subjecting dimethyl maleate to a hydrogenation reaction with hydrogen under the catalysis of a catalyst to obtain crude dimethyl succinate, wherein the catalyst is the catalyst according to the first aspect of the present disclosure or the catalyst prepared by the method according to the second aspect of the present disclosure.

In one or more embodiments, the method further comprises step (b): rectifying the crude dimethyl succinate to obtain dimethyl succinate.

In one or more embodiments, in step (a), a molar ratio of dimethyl maleate to hydrogen is 1:(100-500).

In one or more embodiments, in step (a), a reaction pressure is 1.0-10.0 MPaG.

In one or more embodiments, in step (a), a reaction temperature is 110-230 °C.

In one or more embodiments, in step (a), a gas hourly space velocity of a reactor is 20000-30000 h⁻¹.

In one or more embodiments, in step (a), a conversion rate of dimethyl maleate is ≥ 99.9%.

In one or more embodiments, in step (a), a selectivity to dimethyl succinate is ≥ 99%.

In one or more embodiments, in step (a), a content of γ-butyrolactone in the crude dimethyl succinate is ≤ 5wt%.

In a fourth aspect of the present disclosure, a method for preparing polybutylene succinate is provided, which comprises the following steps:
(A) providing the catalyst according to the first aspect of the present disclosure, or providing the catalyst prepared by the method according to the second aspect of the present disclosure;
(B) subjecting dimethyl maleate to a hydrogenation reaction with hydrogen under the catalysis of the catalyst to prepare dimethyl succinate;
(C) subjecting the dimethyl succinate prepared in step (B) to polymerization reaction with 1,4-butanediol to obtain polybutylene succinate.

The present disclosure has the following beneficial effects:
(1) The present disclosure uses natural minerals with abundant pores as the main carrier of the catalyst. Utilizing the natural minerals' inherent properties such as large mesoporous volume, large specific surface area and excellent adsorption characteristics, rare earth metals and zirconium are introduced to modify the natural mineral carrier. The catalyst of the present disclosure is then prepared by loading the active metal Ni on the modified natural mineral carrier.
   While ensuring the stability of the catalyst's pore structure, acidic properties and mechanical strength, the active metal Ni can maintain uniform dispersion on the carrier surface. The catalyst of the present disclosure exhibits excellent structural stability, as well as high activity and high selectivity in the catalytic preparation of DMS. Particularly, it exhibits excellent performance in the hydrogenation of DMM to produce DMS, which can effectively improve the selectivity to DMS and suppress the formation of GBL, BDO and THF.
(2) The process for preparing DMS of the present disclosure adopts a fixed-bed cold hydrogen circulation system, which can effectively eliminate the hot spot temperature generated during the hydrogenation reaction, accurately control the temperature rise of the bed, and ensure the stability and safety of the reaction. This system also inhibits the occurrence of side reactions, improves the quality and yield of the product, and further effectively reduces energy consumption, enhances production efficiency and lowers production costs.
(3) The method for preparing DMS of the present disclosure can produce high-purity, polymerization-grade DMS products with a purity of ≥ 99.9% and a color number of ≤ 10 AHPA. Compared with PBS products prepared from conventional commercially available succinic acid or succinic anhydride, the PBS products produced using the DMS product of the present disclosure have a higher molecular weight, and their physical property indicators such as terminal carboxyl group, melt flow index and oxidation resistance are more in line with the requirements of downstream products.

### Description of the drawings

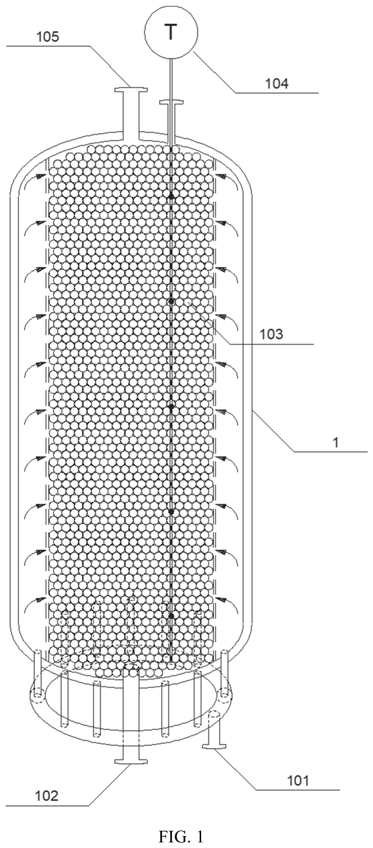

FIG. 1 is a schematic structural diagram of the fixed-bed reactor used in Example 1 of the present disclosure; wherein, 1: reactor; 101: hydrogen inlet; 102: reaction feed inlet; 103: catalyst; 104: multipoint thermometer; 105: reactant outlet.

### Detailed Description

To enable those skilled in the art to understand the features and effects of the present disclosure, the following is a general explanation and definition of the terms and expressions mentioned in the specification and claims. Unless otherwise specified, all technical and scientific terms used herein have the ordinary meanings understood by those skilled in the art with respect to the present disclosure; in case of any conflict, the definitions in this specification shall prevail.

The theories or mechanisms described and disclosed herein, whether correct or incorrect, shall not limit the scope of the present disclosure in any way, that is, the content of the present disclosure may be practiced without being restricted by any specific theory or mechanism.

Herein, the terms "comprise", "include", "contain" and similar expressions cover the meanings of "consisting essentially of" and "consisting of". For example, when the present disclosure discloses that "A comprises B and C", "A consists essentially of B and C" and "A consists of B and C" shall be deemed to have been disclosed herein.

Herein, all features defined in the form of numerical ranges or percentage ranges, such as values, quantities, contents and concentrations, are merely for brevity and convenience. Accordingly, the description of a numerical range or percentage range shall be deemed to have covered and specifically disclosed all possible sub-ranges and individual values (comprising integers and fractions) within the range.

Herein, unless otherwise specified, percentages refer to mass percentages, and ratios refer to mass ratios.

Herein, when describing embodiments or examples, it should be understood that they are not intended to limit the present disclosure to these embodiments or examples. On the contrary, all alternatives, modifications and equivalents of the methods and materials described in the present disclosure may be comprised within the scope defined by the claims.

Herein, for the sake of concise description, not all possible combinations of each technical feature in each embodiment or example are described. Therefore, as long as there is no contradiction in the combination of these technical features, each technical feature in each embodiment or example may be arbitrarily combined, and all possible combinations shall be deemed to be within the scope recorded in this specification.

Herein, rare earth metals refer to metals in Group IIIB of the periodic table, comprising scandium, yttrium, and lanthanides. Specifically, the rare earth metal comprises scandium (Sc), yttrium (Y), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), and lutetium (Lu), preferably one or more of yttrium, lanthanum, cerium, neodymium, samarium, europium, gadolinium, and ytterbium.

The catalyst of the present disclosure is represented by a general formula of Niₓ-Zr_{y}R_{z}P, wherein R represents a rare earth metal, P represents a natural mineral, x represents that a mass fraction of Ni element in the catalyst is x%, and y and z represent that a molar ratio of Zr to R is y:z. In the formula Niₓ-Zr_{y}R_{z}P, x, y, and z do not indicate the content relationship between Ni and Zr, nor the content relationship between Ni and R. In some embodiments, the catalyst is Niₓ-Zr_{y}Ce_{z}P, wherein P is diatomite. In some embodiments, the catalyst is Niₓ-Zr_{y}Ce_{z}P, wherein P is sepiolite. In some embodiments, the catalyst is Niₓ-Zr_{y}Y_{z}P, wherein P is diatomite. In some embodiments, the catalyst is Niₓ-Zr_{y}La_{z}P, wherein P is diatomite.

Herein, since the materials are natural, natural minerals usually contain trace amounts of ionic or organic impurities. To improve the performance of natural minerals and stabilize their properties, those skilled in the art generally optionally subject natural minerals to pretreatment before use. The pretreatment method of natural minerals may be one or more selected from sintering, acid treatment, and alkali treatment. Due to the low content of impurities carried by natural minerals, the mass loss of natural minerals after pretreatment is negligible.

The present disclosure adopts a special reactor structure, which can increase the flow rate of recycled cold hydrogen to a relatively high level without increasing the pressure difference of the reactor. Therefore, the present disclosure provides a reactor applied in the method for preparing dimethyl succinate of the present disclosure. The reactor is a fixed-bed reactor. As shown in FIG. 1, the reactor 1 is composed of nested inner and outer layer structures; the catalyst 103 is loaded in the inner cylinder of the reactor, and the outer cylinder is filled with recycled cold hydrogen. The reaction feed enters the inner cylinder loaded with the catalyst 103 through the reaction feed inlet 102. The reaction liquid after the completion of the reaction is discharged from the reactor 1 through the reactant outlet 105. Hydrogen enters the outer cylinder structure inside the reactor through the hydrogen inlet 101, further reaches the center of the inner cylinder through the catalyst, and then axially passes through the entire catalyst bed. The structure of the reactor of the present disclosure enables the hydrogen entering the reactor 1 to adopt an axial transmission mode, making the fluid passing area much larger than the normal radial area, thereby realizing that the reactor can maintain a low pressure drop under a large flow of recycled cold hydrogen. Preferably, the reactor of the present disclosure is further provided with a multipoint thermometer 104. One or more multipoint thermometers 104 are arranged in the inner cylinder of the reactor through the catalyst 103.

Herein, the reactor bed temperature difference refers to the temperature difference between the temperature at the top of the reactor and the temperature of the bottom discharge. Those skilled in the art can measure the reactor bed temperature difference through a multipoint thermometer (T) inserted from the top of the reactor.

Herein, the reactor bed pressure drop refers to the pressure difference between the pressure at the top of the reactor and the pressure of the bottom of the reactor. Those skilled in the art can measure the reactor bed pressure drop through a pressure transmitter arranged at the top of the reactor and a pressure transmitter arranged at the bottom discharge.

The carrier of the catalyst of the present disclosure is composed of a modifier (comprising zirconium and rare earth metals) and a natural mineral. When applied the catalyst to the hydrogenation of dimethyl maleate to prepare dimethyl succinate, it can produce dimethyl succinate with high yield (high conversion rate of DMS) and high purity (high selectivity to DMS). Therefore, the present disclosure provides a catalyst for catalyzing the reaction of dimethyl maleate with hydrogen to prepare dimethyl succinate.

The catalyst of the present disclosure comprises a composite carrier and active metal Ni loaded on the composite carrier. The composite carrier is composed of a modifier and a natural mineral, wherein the modifier comprises zirconium and a rare earth metal; the natural mineral is one or more selected from diatomite, sepiolite, halloysite, attapulgite, vermiculite and molybdenite; and the rare earth metal is one or more selected from yttrium, lanthanum, cerium, neodymium, samarium, europium, gadolinium and ytterbium.

In some embodiments, the natural mineral is one or more selected from diatomite, sepiolite, halloysite, attapulgite, vermiculite, and molybdenite. Preferably, the natural mineral is one or more selected from diatomite and sepiolite. Using the natural mineral of the present disclosure as one component of the composite carrier enables the prepared catalyst to exhibit high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the rare earth metal is one or more selected from yttrium, lanthanum, cerium, neodymium, samarium, europium, gadolinium, and ytterbium. Preferably, the rare earth metal is one or more selected from yttrium, lanthanum, and cerium. Modifying the natural mineral with the modifier comprising the rare earth metal of the present disclosure to prepare the composite carrier enables the resulting catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the modifier consists of zirconium and a rare earth metal. Modifying the natural mineral with the modifier of the present disclosure to prepare the composite carrier enables the resulting catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the molar ratio of zirconium to the rare earth metal in the modifier is 1:(0.001-0.5), such as 1:0.005, 1:0.008, 1:0.01, 1:0.02, 1:0.05, 1:0.08, 1:0.1, 1:0.2, 1:0.3, 1:0.5, preferably 1:(0.001-0.1), 1:(0.001-0.01), or 1:(0.01-0.1). Controlling the molar ratio of zirconium to the rare earth metal in the modifier within the range defined by the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the content of zirconium element is 0.01-30wt% based on the weight of the natural mineral, such as 0.05wt%, 0.1wt%, 0.2wt%, 0.5wt%, 1wt%, 2wt%, 3wt%, 4wt%, 5wt%, 7wt%, 9wt%, 11wt%, 13wt%, 15wt%, 17wt%, preferably 0.5-20wt%, 1-5wt%, 5-10wt%, or 5-20wt%. Controlling the content of zirconium element within the range defined by the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the content of the active metal Ni in the catalyst is 1-40wt%, such as 2wt%, 5wt%, 8wt%, 10wt%, 13wt%, 15wt%, 18wt%, 25wt%, 30wt%, 35wt%, 40wt%, preferably 5-25wt%, 10-20wt%, or 5-10wt%. Controlling the content of Ni in the catalyst within the range defined by the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

The present disclosure also provides a composite carrier, which is formed by compounding a modifier and a natural mineral. The modifier and natural mineral are as described in any one of the embodiments herein. The catalyst prepared using this composite carrier has excellent structural stability, as well as high activity and high selectivity in the catalytic preparation of DMS. The catalyst prepared with this composite carrier exhibits excellent performance in the hydrogenation of DMM to DMS, which can effectively improve the selectivity to DMS and suppress the formation of GBL, BDO, and THF.

The present disclosure also provides a method for preparing the composite carrier, which comprises the following steps:
(1) preparing a modifier from an aqueous solution containing a zirconium salt and a rare earth metal salt by a high-pressure hydrothermal method;
(2) forming a precipitate from the modifier and the natural mineral in a dispersant, filtering and collecting the precipitate, drying and calcining the precipitate to obtain a composite carrier;
steps (1) and (2) are as described in any one of the embodiments herein.

In some embodiments, the specific surface area of the composite carrier is 150-450 m²/g, such as 155 m²/g, 160 m²/g, 165 m²/g, 170 m²/g, 175 m²/g, 177 m²/g, 180 m²/g, 185 m²/g, 187 m²/g, 188 m²/g, 200 m²/g, 220 m²/g, 250 m²/g, 280 m²/g, 320 m²/g, 350 m²/g, 400 m²/g, preferably 150-250 m²/g, 170-190 m²/g, or 177-188 m²/g. Preparing the catalyst of the present disclosure using the composite carrier with the specific surface area defined herein enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the total pore volume of the composite carrier is 0.15-1.5 cm³/g, such as 0.2 cm³/g, 0.25 cm³/g, 0.3 cm³/g, 0.35 cm³/g, 0.38 cm³/g, 0.4 cm³/g, 0.45 cm³/g, 0.5 cm³/g, 0.6 cm³/g, 0.7 cm³/g, 0.8 cm³/g, 1.0 cm³/g, 1.3 cm³/g, preferably 0.3-1.0 cm³/g or 0.35-0.5 cm³/g. Preparing the catalyst of the present disclosure using the composite carrier with the total pore volume defined herein enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, the average pore diameter of the composite carrier is 5-30 nm, such as 8 nm, 10 nm, 12 nm, 15 nm, 18 nm, 20 nm, 22 nm, 25 nm, 28 nm, preferably 8-20 nm, 10-12 nm, or 10-15 nm. Preparing the catalyst of the present disclosure using the composite carrier with the average pore diameter defined herein enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

The inventors have found that natural minerals modified with rare earth metals and zirconium can obtain a suitable specific surface area and pore structure. When the modified natural mineral is used as a composite carrier, the catalyst prepared by loading the active metal Ni on this composite carrier exhibits excellent performance in the hydrogenation of DMM to produce DMS, possessing high catalytic activity and high selectivity. It can effectively improve the selectivity to DMS and suppress the formation of GBL, BDO, and THF.

The present disclosure provides a method for preparing the catalyst of the present disclosure, which comprises the following steps:
(1) preparing a modifier from an aqueous solution containing a zirconium salt and a rare earth metal salt by a high-pressure hydrothermal method;
(2) forming a precipitate from the modifier and the natural mineral in a dispersant, filtering and collecting the precipitate, drying and calcining the precipitate to obtain a composite carrier;
(3) impregnating the composite carrier in an aqueous solution containing a nickel salt, filtering off the solution, drying and calcining to obtain a catalyst precursor;
(4) subjecting the catalyst precursor to a reduction reaction in a reducing gas to obtain the catalyst.

In some embodiments, in step (1), the zirconium salt is one or more selected from zirconium nitrate, zirconium chloride and zirconium alkoxide. Preferably, the zirconium salt is one or more selected from ZrOCl₂, ZrO(NO₃)₂ and ZrCl₄, such as ZrO(NO₃)₂.

In some embodiments, in step (1), the rare earth metal salt is one or more selected from Gd(NO₃)₃·6H₂O, Yb(NO₃)₃·5H₂O, Y(NO₃)₃·4H₂O, La(NO₃)₃·6H₂O, Ce(NO₃)₃·6H₂O, Nd(NO₃)₃·6H₂O, Sm(NO₃)₃·6H₂O, Eu(NO₃)₃·5H₂O and chlorides of the rare earth metals. Preferably, the rare earth metal salt is one or more selected from Ce(NO₃)₃·6H₂O, La(NO₃)₃·6H₂O and Y(NO₃)₃·4H₂O.

Using the zirconium salt and rare earth metal salt of the present disclosure to prepare the catalyst of the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, in step (1), the concentration of the zirconium salt in the aqueous solution is 2-2.5 mol/L, such as 2 mol/L, 2.2 mol/L, 2.3 mol/L, 2.5 mol/L, or a range between any two values.

In some embodiments, in step (1), the molar ratio of zirconium to the rare earth metal in the aqueous solution is 1:(0.001-0.5), such as 1:0.005, 1:0.008, 1:0.01, 1:0.02, 1:0.05, 1:0.08, 1:0.1, 1:0.2, 1:0.3, 1:0.5, preferably 1:(0.001-0.1), 1:(0.001-0.01), 1:(0.01-0.1), 1:(0.01-0.05) or 1:(0.01-0.02).

In some embodiments, in step (1), the reaction pressure of the high-pressure hydrothermal method is 1.0-3.0 MPa, such as 1.0 MPa, 1.5 MPa, 2.0 MPa, 2.5 MPa, 3.0 MPa, preferably 2.0-3.0 MPa.

In some embodiments, in step (1), the reaction temperature of the high-pressure hydrothermal method is 110-150 °C, such as 115 °C, 120 °C, 125 °C, 130 °C, 135 °C, 140 °C, 145 °C, 150 °C, preferably 120-130 °C or 130-140 °C.

In some embodiments, in step (1), the reaction time of the high-pressure hydrothermal method is 3-24 h, such as 3 h, 5 h, 6 h, 8 h, 10 h, 15 h, 18 h, 20 h, preferably 4-6h, 6-18 h or 6-10 h.

In step (1), the modifier is prepared using the concentration of the zirconium salt in the aqueous solution, the molar ratio of zirconium to the rare earth metal in the aqueous solution, the reaction pressure of the high-pressure hydrothermal method, the reaction temperature of the high-pressure hydrothermal method, and the reaction time of the high-pressure hydrothermal method, all as defined in the present disclosure. Compounding the obtained modifier with the natural mineral can afford a composite carrier suitable for the catalyst of the present disclosure. Using the composite carrier to prepare the catalyst of the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

The present disclosure provides a method for preparing the modifier described herein, which comprises step (1): preparing a modifier from an aqueous solution containing a zirconium salt and a rare earth metal salt by a high-pressure hydrothermal method; step (1) is as defined in any one of the embodiments herein.

In some embodiments, in step (2), the modifier is added in an amount such that a content of zirconium element in the modifier is 0.01-30wt% based on the weight of the natural mineral, such as 0.05wt%, 0.1wt%, 0.2wt%, 0.5wt%, 1wt%, 2wt%, 3wt%, 4wt%, 5wt%, 7wt%, 9wt%, 11wt%, 13wt%, 15wt%, 17wt%, preferably 0.5-20wt%, 1-5wt%, 3-8wt%, 5-10wt%, or 5-20wt%.

In some embodiments, in step (2), the dispersant is one or more selected from methanol, ethanol, and water. In some embodiments, the dispersant is a mixture of ethanol and water; preferably, the volume ratio of ethanol to water is (0.1-10):1, such as 0.1:1, 0.2:1, 0.5:1, 1:1, 3:1, 5:1, 8:1, 10:1, preferably (5-8):1, (3-5):1, or (3-8):1.

In some embodiments, in step (2), 100-500 g of the natural mineral is added per liter of the dispersant, such as 100 g, 150 g, 200 g, 250 g, 300 g, 330 g, 350 g, 380 g, 400 g, 450 g, preferably 300-400 g or 330-350 g.

In step (2), the conditions for forming the precipitate are: dispersing the modifier and the natural mineral under stirring with heating, followed by standing under heating until a precipitate is formed. In some embodiments, the stirring and standing are each independently carried out at 50-90 °C, preferably at 50-80 °C, 60-80 °C, or 80-90 °C. Preferably, the stirring is carried out for 2-12 h, such as 2 h, 4 h, 6 h, 8 h, 10 h, 12 h, 4-6 h, 2-4 h, or 2-6 h. Preferably, the standing is carried out for 4-48 h, such as 8 h, 12 h, 18 h, 24 h, 30 h, 35 h, 40 h, 12-24 h, 24-30 h, or 20-30 h.

In some embodiments, in step (2), the drying temperature is 100-150 °C, such as 110 °C, 120 °C, 130 °C, 140 °C, 150 °C, preferably 100-120 °C, 120-130 °C, or 120-150 °C.

In some embodiments, in step (2), the drying time is 2-48 h, such as 4 h, 8 h, 12 h, 15 h, 18 h, 24 h, 30 h, 36 h, 42 h, preferably 5-12 h, 2-8 h, or 8-24 h.

In some embodiments, in step (2), the calcination temperature is 150-800 °C, such as 150 °C, 200 °C, 250 °C, 300 °C, 350 °C, 400 °C, 450 °C, 500 °C, 550 °C, 600 °C, 650 °C, 700 °C, 750 °C, 800 °C, preferably 200-650 °C, 200-450 °C, 450-800 °C, 450-500 °C, or 400-450 °C.

In some embodiments, in step (2), the calcination time is 0.5-12 h, such as 1 h, 2 h, 4 h, 6 h, 8 h, 10 h, preferably 1-8 h, 2-4 h, or 4-12 h.

Prior to performing step (2), the natural mineral is subjected to heat treatment and/or chemical treatment.

The temperature of the heat treatment is 300-800 °C, preferably 500-600 °C. The time of the heat treatment is 0.5-5 h, preferably 2-4 h. The heat treatment is carried out in an air atmosphere.

The chemical treatment is soaking the natural mineral in an acid solution. The acid may be one or more selected from sulfuric acid, hydrochloric acid and phosphoric acid; preferably sulfuric acid. The concentration of hydrogen ions in the acid solution is 0.1-0.8 mol/L, such as 0.2 mol/L, 0.4 mol/L, 0.6 mol/L, 0.2-0.4 mol/L, or 0.1-0.2 mol/L. In some embodiments, the acid solution is an aqueous solution of acid. In some embodiments, based on the total mass of the natural mineral and the acid solution, the content of the natural mineral is 1-80wt%, preferably 1-60wt%, 30-60wt%, 30-40wt%, or 20-30wt%. The chemical treatment may be carried out at 30-150 °C, preferably 50-120 °C, 50-80 °C, or 50-60 °C. The chemical treatment is performed for 0.5-24 h, preferably 1-16 h or 5-8 h. Preferably, the chemically treated natural mineral is washed to a pH of 6-7. Preferably, the chemically treated and washed natural mineral is dried.

In some embodiments, prior to performing step (2), the natural mineral is sequentially subjected to the above heat treatment and chemical treatment.

In step (2), the composite carrier is prepared using the addition amount of the modifier, the type of the dispersant, the addition amount of the natural mineral, the conditions for forming the precipitate, the drying temperature, the drying time, the calcination temperature, the calcination time, and the heat treatment and/or chemical treatment method for the natural mineral, all as defined in the present disclosure. Using the composite carrier to prepare the catalyst of the present disclosure enables the prepared catalyst to have high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, in step (3), the nickel salt is one or more selected from nickel nitrate, nickel chloride and nickel sulfate, preferably nickel nitrate.

In some embodiments, in step (3), the aqueous solution containing the nickel salt further comprises an organic acid. The organic acid may be one or more selected from formic acid, acetic acid, oxalic acid and citric acid, preferably citric acid. Preferably, the aqueous solution containing the nickel salt contains 0.9-35 vol% of the organic acid, such as 1 vol%, 2 vol%, 3 vol%, 5 vol%, 7 vol%, 9 vol%, 11 vol%, 15 vol%, 20 vol%, 25 vol%, 30 vol%, preferably 5-20 vol% or 5-10 vol%.

In some embodiments, the concentration of the nickel salt in the aqueous solution containing the nickel salt is 0.01-2.0 g/mL, such as 0.05 g/mL, 0.08 g/mL, 0.1 g/mL, 0.3 g/mL, 0.5 g/mL, 0.8 g/mL, 0.9 g/mL, 1.0 g/mL, 1.3 g/mL, 1.5 g/mL, 1.8 g/mL, preferably 0.2-0.5 g/mL, 0.5-0.9 g/mL or 0.8-0.9 g/mL.

In some embodiments, the impregnation time is 20-240 min, such as 20 min, 30 min, 50 min, 80 min, 100 min, 120 min, 150 min, 180 min, 210 min, 230 min, preferably 20-120 min, 50-120 min or 80-120 min.

In step (3), parameters such as the volume of the solvent used in the impregnation, the content of the nickel salt in the solvent, the content of the organic acid in the impregnation solution, and the amount of the composite carrier can all be adjusted according to the content of Ni in the catalyst to be prepared. Those skilled in the art can rationally design the above parameters according to the mass fraction of Ni in the catalyst to be prepared.

In some embodiments, in step (3), the drying temperature is 80-120 °C, such as 80 °C, 90 °C, 100 °C, 110 °C, 120 °C, preferably 100-120 °C.

In some embodiments, in step (3), the drying time is 2-12 h, such as 2 h, 4 h, 6 h, 8 h, 10 h, 12 h, preferably 4-12 h or 4-8 h.

In some embodiments, in step (3), the calcination temperature is 150-550 °C, such as 150 °C, 200 °C, 250 °C, 300 °C, 350 °C, 400 °C, 450 °C, 500 °C, 550 °C, preferably 170-550 °C, 300-550 °C or 500-550 °C.

In some embodiments, in step (3), the calcination time is 0.5-50 h, such as 1 h, 2 h, 4 h, 8 h, 12 h, 15 h, 20 h, 24 h, 36 h, 48 h, preferably 1-24 h, 8-24 h or 2-8 h.

In step (3), the catalyst precursor is prepared using the nickel salt, the aqueous solution containing the nickel salt, the impregnation time, the drying temperature, the drying time, the calcination temperature, and the calcination time, all as defined in the present disclosure. The catalyst of the present disclosure obtained by further reducing the catalyst precursor has high activity and high selectivity in the catalytic preparation of DMS; the catalyst exhibits excellent performance in the hydrogenation of DMM to produce DMS, which can effectively improve the selectivity to DMS and suppress the formation of GBL, BDO, and THF.

In some embodiments, in step (4), the reducing gas comprises hydrogen and a protective gas. The protective gas is one or more selected from nitrogen, helium and argon, preferably nitrogen. In the reducing gas, the molar ratio of the protective gas to hydrogen is 1:(0.01-1), such as 1:0.01, 1:0.02, 1:0.05, 1:0.08, 1:0.1, 1:0.2, 1:0.3, 1:0.5, 1:0.8, 1:0.9, preferably 1:(0.01-0.1), 1:(0.05-1) or 1:(0.05-0.1).

In some embodiments, the temperature of the reduction reaction is 130-650 °C, such as 150 °C, 200 °C, 300 °C, 350 °C, 400 °C, 450 °C, 500 °C, 550 °C, 600 °C, 650 °C, 350-400 °C or 300-350 °C.

In some embodiments, the system of the reduction reaction is heated to a set temperature at a heating rate of not more than 10 °C/h.

In some embodiments, after the temperature of the system for the reduction reaction reaches the set value, the system is maintained for 4-48 h, such as 4 h, 8 h, 12 h, 18 h, 24 h, 30 h, 36 h, 42 h, preferably 6-24 h or 12-30 h.

The inventors have found that the catalyst prepared by the method of the present disclosure has excellent performance, high catalytic activity and high selectivity in the hydrogenation of DMM to produce DMS, and can effectively improve the selectivity to DMS and suppress the formation of GBL, BDO and THF.

The catalyst of the present disclosure is prepared by employing the type of the reducing gas, the temperature of the reduction reaction, the heating rate of the reduction reaction, and the reaction time of the reduction reaction as defined in the present disclosure. The catalyst thus obtained has high activity and high selectivity in the catalytic preparation of DMS. The catalyst exhibits excellent performance in the hydrogenation of DMM to produce DMS, which can effectively improve the selectivity to DMS and suppress the formation of GBL, BDO, and THF.

In some embodiments, the catalyst is stored under air isolation conditions under protection of a protective gas (e.g., one or two of nitrogen and argon) or liquid sealing (e.g., using one or two of water and ethanol).

The present disclosure provides a method for preparing dimethyl succinate, which comprises step (a): subjecting dimethyl maleate to a hydrogenation reaction with hydrogen under the catalysis of a catalyst to obtain crude dimethyl succinate, wherein the catalyst is the catalyst of the present disclosure or the catalyst prepared by the method of the present disclosure.

In some embodiments, the method further comprises step (b): rectifying the crude dimethyl succinate to obtain dimethyl succinate.

In the present disclosure, the reactor shown in FIG. 1 is used for the preparation of dimethyl succinate, so that the reactor bed temperature difference and the reactor bed pressure drop of the reactor during the reaction can be controlled. In the method of the present disclosure, the bed temperature rise is controlled by a high-flow cold hydrogen circulation, which can eliminate the hot spot temperature in the catalyst bed, suppress the occurrence of side reactions, and effectively improve the selectivity and conversion rate of dimethyl succinate.

In some embodiments, the reactor bed temperature difference (Δt) can be controlled to ≤ 1 °C, and as low as ≤ 0.1 °C.

In some embodiments, the reactor bed pressure drop (ΔP) can be controlled to ≤ 0.2 MPa, and as low as ≤ 0.1 MPa.

Using the reactor shown in FIG. 1 of the present disclosure to prepare dimethyl succinate and controlling the reactor bed temperature difference and reactor bed pressure drop within the ranges defined herein can suppress the occurrence of side reactions and effectively improve the selectivity and conversion rate of dimethyl succinate.

In step (a), the molar ratio of dimethyl maleate to hydrogen is 1:(100-500), such as 1:100, 1:150, 1:200, 1:250, 1:300, 1:350, 1:400, 1:450, 1:500, preferably 1:(300-400), 1:(200-300), 1:(300-350), 1:(250-300), 1:(100-300), or 1:(300-500).

In step (a), the reaction pressure is 1.0-10.0 MPaG, such as 1.0 MPaG, 2 MPaG, 3 MPaG, 5 MPaG, 8 MPaG, 9 MPaG, preferably 1-5 MPaG, 3-8 MPaG, or 3-5 MPaG.

In step (a), the reaction temperature is 110-230 °C, such as 110 °C, 120 °C, 130 °C, 150 °C, 170 °C, 190 °C, 210 °C, 230 °C, preferably 125-210 °C, 110-190 °C, 150-230 °C, or 190-230 °C.

In step (a), the gas hourly space velocity of the reactor is 20000-30000 h⁻¹, such as 21000 h⁻¹, 22000 h⁻¹, 23000 h⁻¹, 24000 h⁻¹, 25000 h⁻¹, 27000 h⁻¹, 29000 h⁻¹, preferably 21000-25000 h⁻¹ or 23000-24000 h⁻¹.As used herein, the gas hourly space velocity of the reactor refers to the standard gas hourly space velocity of the reactor, which means the space velocity of the feed gas under standard conditions.

In step (a), the conversion rate of dimethyl maleate is ≥ 99.9%.

In step (a), the selectivity to dimethyl succinate is ≥ 99%, such as ≥ 99.1%, ≥ 99.2%, ≥ 99.3%, ≥ 99.4%, ≥ 99.5%, ≥ 99.6%, ≥ 99.7%, ≥ 99.8%.

In step (a), the content of γ-butyrolactone in the crude dimethyl succinate is ≤ 5wt%, such as ≤ 4wt%, ≤ 3wt%, ≤ 2wt%, ≤ 1wt%, ≤ 0.5wt%, ≤ 0.1wt%, ≤ 0.05wt%, ≤ 0.01wt%, ≤ 0.005wt%, ≤ 0.003wt%, ≤ 0.002wt%, ≤ 0.001wt%.

In step (a), using the conditions of the molar ratio of dimethyl maleate to hydrogen, the reaction pressure, the reaction temperature, and the gas hourly space velocity of the reactor as defined herein to prepare dimethyl succinate can effectively improve the conversion rate of dimethyl maleate and the selectivity to dimethyl succinate, and control the content of γ-butyrolactone in dimethyl succinate.

In step (b), the rectification is three-stage rectification. The three-stage rectification comprises a first-stage rectification. The operating pressure of the first-stage rectification is 5-50 kPaA, preferably 10-30 kPaA, 10-20 kPaA, or 20-30 kPaA. The operating temperature of the first-stage rectification is 130-180 °C, such as 135 °C, 140 °C, 145 °C, 150 °C, 155 °C, 160 °C, 165 °C, 170 °C, 175 °C, preferably 145-175 °C, 130-170 °C, or 140-165 °C. The three-stage rectification comprises a second-stage rectification. The operating pressure of the second-stage rectification is 0.1-10 kPaA, such as 0.2 kPaA, 0.5 kPaA, 1 kPaA, 2 kPaA, 3 kPaA, 4 kPaA, 5 kPaA, 8 kPaA, preferably 0.5-6.5 kPaA, 1-5 kPaA, or 1-3 kPaA. The operating temperature of the second-stage rectification is 130-200 °C, such as 135 °C, 140 °C, 145 °C, 150 °C, 155 °C, 165 °C, 170 °C, 172 °C, 178 °C, 190 °C, preferably 135-185 °C, 140-180 °C, or 150-175 °C. The three-stage rectification comprises a third-stage rectification. The operating pressure of the third-stage rectification is 1-30 kPaA, such as 2 kPaA, 5 kPaA, 8 kPaA, 10 kPaA, 15 kPaA, 20 kPaA, 25 kPaA, 30 kPaA, preferably 1-20 kPaA, 10-15 kPaA, or 5-10 kPaA. The operating temperature of the third-stage rectification is 110-170 °C, such as 120 °C, 130 °C, 135 °C, 140 °C, 150 °C, 160 °C, 168 °C, 170 °C, preferably 125-165 °C, 130-170 °C, or 150-168 °C.

The purity of dimethyl succinate obtained in step (b) is ≥ 95wt%, such as ≥ 96wt%, ≥ 97wt%, ≥ 98wt%, ≥ 99wt%, ≥ 99.1wt%, ≥ 99.2wt%, ≥ 99.3wt%, ≥ 99.4wt%, ≥ 99.5wt%, ≥ 99.6wt%, ≥ 99.7wt%, ≥ 99.8wt%, ≥ 99.9wt%.

The color number of dimethyl succinate obtained in step (b) is ≤ 10 APHA, such as 10 APHA, 9 APHA, 8 APHA, 7 APHA, 6 APHA, 5 APHA, 4 APHA, 3 APHA, 2 APHA, 1 APHA, preferably 2-8 APHA or 3-5 APHA.

In step (b), the crude dimethyl succinate obtained in step (a) is further rectified by using the three-stage rectification conditions defined in the present disclosure, thereby obtaining a dimethyl succinate product with high purity and low color number.

The present disclosure also provides a method for preparing polybutylene succinate, comprising the following steps:
(A) providing the catalyst of the present disclosure, or a catalyst prepared by the method of the present disclosure;
(B) subjecting dimethyl maleate to a hydrogenation reaction with hydrogen under the catalysis of the catalyst to prepare dimethyl succinate;
(C) subjecting the dimethyl succinate prepared in step (B) to polymerization reaction with 1,4-butanediol to obtain polybutylene succinate.

The catalyst of the present disclosure, the preparation method of the catalyst of the present disclosure, and the method for preparing dimethyl succinate by hydrogenation of dimethyl maleate are as described in any one of the embodiments herein.

In some embodiments, in step (C), the molar ratio of dimethyl succinate to 1,4-butanediol is 1:(1-1.5), such as 1:1.1, 1:1.2, 1:1.3, 1:1.4, 1:1.5, preferably 1:(1-1.3) or 1:(1-1.2).

Step (C) comprises the steps of:
(C1) subjecting dimethyl succinate and 1,4-butanediol to transesterification;
(C2) subjecting a reaction solution obtained after completion of the reaction in step (C1) to pre-polycondensation;
(C3) subjecting a reaction solution obtained after completion of the reaction in step (C2) to final polycondensation.

In some embodiments, the reaction pressure of the transesterification is 5-20 kPaG, such as 5 kPaG, 8 kPaG, 10 kPaG, 12 kPaG, 15 kPaG, 18 kPaG, 20 kPaG, preferably 5-10 kPaG, 10-20 kPaG, or 5-15 kPaG.

In some embodiments, the reaction temperature of the transesterification is 140-200 °C, such as 140 °C, 150 °C, 160 °C, 170 °C, 180 °C, 190 °C, 200 °C, preferably 140-160 °C or 160-200 °C.

In some embodiments, the reaction time of the transesterification is 3-10 h, such as 3 h, 4 h, 6 h, 8 h, 10 h, preferably 3-6 h, 6-10 h, or 3-8 h.

In some embodiments, the transesterification is terminated when the content of dimethyl succinate in the reaction system is detected to be below 500 ppm.

In some embodiments, the pre-polycondensation is carried out under the catalysis of a polymerization catalyst. Preferably, the polymerization catalyst is a Ti-containing catalyst, preferably tetraisopropyl titanate (TtiPO, CAS: 546-68-9).

In some embodiments, the molar ratio of the polymerization catalyst to dimethyl succinate fed in step (C1) is 1:(300-500), such as 1:320, 1:340, 1:360, 1:380, 1:400, 1:420, 1:440, 1:480, preferably 1:(400-500), 1:(300-400), 1:(350-400), or 1:(400-450).

In some embodiments, the reaction temperature of the pre-polycondensation is 180-250 °C, such as 190 °C, 200 °C, 210 °C, 220 °C, 230 °C, 240 °C, preferably 180-210 °C or 210-250 °C.

In some embodiments, the reaction pressure of the pre-polycondensation is 0.5-2 kPaA, such as 0.8 kPaA, 1 kPaA, 1.2 kPaA, 1.4 kPaA, 1.6 kPaA, 1.8 kPaA, preferably 0.5-1 kPaA or 1-2 kPaA.

In some embodiments, the reaction time of the pre-polycondensation is 2-5 h, such as 3 h, 4 h, e.g., 2-3 h or 3-4 h.

In some embodiments, the reaction pressure of the final polycondensation is 50-80 PaA, such as 55 PaA, 60 PaA, 65 PaA, 70 PaA, 75 PaA, preferably 60-80 PaA or 65-80 PaA.

In some embodiments, the reaction temperature of the final polycondensation is 200-250 °C, such as 205 °C, 210 °C, 215 °C, 220 °C, 225 °C, 230 °C, 235 °C, 240 °C, preferably 225-250 °C, 210-240 °C, or 220-230 °C.

In some embodiments, the reaction time of the final polycondensation is 1-5 h, such as 2 h, 3 h, 4 h, e.g., 2-3 h or 3-4 h.

In some embodiments, the final polycondensation is completed when the viscosity of the reaction system no longer increases (i.e., the viscosity of the reaction system remains stable).

In some embodiments, prior to the transesterification, dimethyl succinate and 1,4-butanediol are fully dissolved, for example, by heating. The heating may be carried out at 100-150 °C (e.g., 140-150 °C) for 0.5-5 h (e.g., 0.5-1 h or 1-2 h).

The terminal carboxyl group content of the polybutylene succinate prepared by the method of the present disclosure is 30-80 mol/t, such as 35 mol/t, 40 mol/t, 45 mol/t, 50 mol/t, 55 mol/t, 60 mol/t, 65 mol/t, 70 mol/t, 75 mol/t, preferably 35-80 mol/t, 40-80 mol/t, 45-80 mol/t, or 45-50 mol/t.

The melt flow index of the polybutylene succinate prepared by the method of the present disclosure is 5.0-8.0 g/min, such as 5.5 g/min, 6.0 g/min, 6.2 g/min, 6.5 g/min, 7.0 g/min, 7.5 g/min, 8.0 g/min, preferably 6.0-8.0 g/min, 6.2-8.0 g/min, or 6.2-7.0 g/min.

The oxidation induction time of the polybutylene succinate prepared by the method of the present disclosure is 60-150 min, such as 65 min, 70 min, 75 min, 80 min, 85 min, 90 min, 95 min, 100 min, 105 min, 110 min, 115 min, 116 min, 120 min, 125 min, 130 min, 135 min, 140 min, 145 min, preferably 65-116 min, 116-150 min, or 90-120 min.

The oxidation induction temperature of the polybutylene succinate prepared by the method of the present disclosure is 250-350 °C, such as 255 °C, 260 °C, 265 °C, 270 °C, 275 °C, 280 °C, 290 °C, 300 °C, 310 °C, 320 °C, 330 °C, 340 °C, preferably 280-320 °C, 250-300 °C, or 290-340 °C.

When polybutylene succinate is prepared using the molar ratio of dimethyl succinate to 1,4-butanediol, the transesterification conditions, the pre-polycondensation conditions, and the final polycondensation conditions as defined herein, the physical properties of the obtained polybutylene succinate, comprising terminal carboxyl group content, melt flow index, and oxidation resistance, are more consistent with the requirements of downstream products.

Owing to the advantages of the catalyst of the present disclosure, as well as its preparation method and uses, the catalyst of the present disclosure can be widely used in fields such as petrochemical engineering, catalyst research and development, environmental protection, and energy conservation.

In the field of petrochemical engineering, the present disclosure provides a novel Ni-based catalyst with a modified composite carrier for the gas-phase hydrogenation of dimethyl maleate (DMM) to produce dimethyl succinate (DMS), and a production process for the catalytic hydrogenation of DMM to DMS using the catalyst. As described herein, the process for the gas-phase hydrogenation of DMM to DMS catalyzed by the catalyst of the present disclosure features high DMM conversion, high DMS selectivity, and low production cost, and can significantly reduce side reactions and the formation of by-products during the DMM hydrogenation.

The present disclosure provides an optimized hydrogenation reaction process. The process employs a reactor with a special structure, which enables the reactor to maintain a low pressure drop even at a high hydrogen circulation rate and eliminates hot spots in the catalyst bed. The process of the present disclosure has the advantages of high product purity, low energy consumption, and easy controllability, which can greatly improve the yield and quality of DMS, reduce production costs, and promote the rapid development of the degradable plastic PBS industry.

In the field of environmental protection and energy conservation, the method of the present disclosure adopts cold hydrogen circulation to strictly control the bed temperature rise and avoid hot spots, thereby improving the safety and stability of the reaction, reducing energy waste, lowering carbon emissions during production, and facilitating environmental protection and sustainable development.

In summary, the method for preparing DMS of the present disclosure is characterized by high selectivity and low energy consumption, which is conducive to reducing resource waste, improving resource utilization efficiency, and achieving the goals of environmental protection and energy conservation.

The present disclosure will be further illustrated with reference to specific examples. It should be understood that these examples are only used to illustrate the present disclosure and not to limit the scope of the present disclosure. The experimental methods in the following examples that do not specify specific conditions are generally carried out in accordance with conventional conditions or the conditions recommended by the manufacturer. Unless otherwise stated, percentages and parts are by weight.

As used herein, the specific surface area of the composite carrier is determined as follows: measured by the BET method using a Microtrac BELSORP-mini x specific surface area analyzer (Japan), by the static volumetric method, with degassing at 300 °C for 8 h, and the specific surface area is calculated using the Brunauer-Emmett-Teller (BET) equation.

As used herein, the total pore volume and average pore diameter of the composite carrier are both calculated by the BJH (Barrett-Joyner-Halenda) model. The average pore diameter of the composite carrier is obtained by dividing the corresponding pore volume by the corresponding specific surface area. The calculation formula is:
Average pore diameter *Pₐᵥₑᵣ =k*× Total pore volume / Specific surface area, wherein *k* is taken as the value for a cylindrical pore, k = 4.

As used herein, the Zr-rare earth modifier is prepared by a high-pressure hydrothermal method. Before the high-pressure hydrothermal treatment, the molar ratio of zirconium to rare earth elements charged in the aqueous solvent is a:b; after the high-pressure hydrothermal treatment, the molar ratio of zirconium to rare earth elements in the obtained Zr-rare earth modifier is approximately a:b.

### Preparation Example 1: Preparation of Catalyst 1

### (1) Preparation of Zr-Rare Earth Zr₁Ce_{0.01} Modifier

An aqueous solution of ZrO(NO₃)₂ with a concentration of 2.0 mol/L is prepared. Ce(NO₃)₃·6H₂O is added at a molar ratio of Zr to Ce of 1:0.01 in the aqueous solution. After uniform mixing, the mixture is transferred into an autoclave, pressurized to 3.0 MPa, and reacted at 130 °C for 6 h to obtain the Zr-rare earth Zr₁Ce_{0.01} modifier. The system is cooled to atmospheric pressure and room temperature, and the solid (the obtained Zr-rare earth Zr₁Ce_{0.01} modifier) is collected by filtration and reserved for use.

### (2) Pretreatment of Diatomite (P)

A certain amount of commercially available natural diatomite (Chuanyi Diatomite Ore, Changbai Diatomite Deposit, Jilin) is weighed. Heat treatment is first carried out, and calcination is performed at 600 °C for 2 h in an air atmosphere to remove organic matters contained in the natural diatomite ore. The heat-treated diatomite is ground into uniform powder. Subsequently, the heat-treated diatomite powder is subjected to chemical treatment in sulfuric acid to remove alkali ions therein. The uniformly ground powder is suspended in dilute sulfuric acid with a concentration of 0.2 mol/L. The solid content of the suspension is 30wt%, and continuous stirring is performed at 50 °C for 8 h. Separation is then carried out by suction filtration, washing is performed with deionized water, the filter cake is dried at 120 °C to constant weight, and grinding into powder is conducted.

### (3) Preparation of Composite Carrier ZrₓR_{y}P

100 g of the diatomite treated in step (2) is weighed, and 300 mL of a mixed solution of ethanol and water (volume ratio 5:1) is added. Stirring is carried out until a suspension is formed. The modifier prepared in step (1) is added at a zirconium content of 5wt% (based on the amount of natural diatomite). Continuous stirring is performed at 80 °C for 4 h, followed by standing at 80 °C for 24 h without stirring until a stable colloidal precipitate is formed. The solvent is removed by filtration, and the precipitate is dried at 120 °C for 8 h in an air atmosphere. Finally, the dried carrier sample is calcined at 450 °C for 4 h to obtain the composite carrier. The obtained composite carrier is determined to have a specific surface area (BET) of 180 m²/g, a total pore volume of 0.5 cm³/g, and an average pore diameter of 12 nm.

### (4) Preparation of Catalyst Precursor

The active metal Ni is loaded on the composite carrier by an impregnation method. First, 54.2 g of Ni(NO₃)₂·6H₂O is weighed and dissolved into 200 mL of aqueous nickel salt solution. Citric acid is added to the prepared aqueous nickel salt solution at a volume ratio of aqueous nickel salt solution to citric acid of 1:0.1. The composite carrier is impregnated with the mixed solution of aqueous nickel salt solution and citric acid. The amount of the composite carrier is selected according to the Ni loading on the catalyst to be prepared in step (5). Standing is performed for 120 min, excess solution is removed by filtration, drying is carried out at 120 °C for 4 h, and calcination is performed at 550 °C for 8 h in an air atmosphere to obtain the catalyst precursor.

### (5) Preparation of Catalyst with 10wt% Ni Loading

The catalyst precursor prepared in step (4) is reduced under a reducing gas to obtain a catalyst with metallic Ni as the active center. The reducing gas is a mixture of nitrogen and hydrogen at a molar ratio of nitrogen to hydrogen of 1:0.05. Temperature is raised to 350 °C by programmed heating at a heating rate of no more than 10 °C/h. After reaching the set temperature, holding is performed for 24 h, and natural cooling to room temperature is carried out to obtain catalyst Ni₁₀-Zr₁R_{0.01}P with a Ni content of 10wt%.The obtained catalyst is sealed with deionized water and stored under air isolation.

### Preparation Example 2: Preparation of Catalyst 2

The procedure of Preparation Example 1 is repeated, with the only difference that diatomite is replaced by commercially available sepiolite powder (from Xiangtan Sepiolite Technology Co., Ltd.).

The obtained composite carrier is determined to have a specific surface area of 187 m²/g, a total pore volume of 0.42 cm³/g, and an average pore diameter of 10 nm.

### Preparation Example 3: Preparation of Catalyst 3

The procedure of Preparation Example 1 is repeated, with the only difference that Ce(NO₃)₃·6H₂O is replaced by Y(NO₃)₃·4H₂O.

The obtained composite carrier is determined to have a specific surface area of 188 m²/g, a total pore volume of 0.40 cm³/g, and an average pore diameter of 12 nm.

### Preparation Example 4: Preparation of Catalyst 4

The procedure of Preparation Example 1 is repeated, with the only difference that Ce(NO₃)₃·6H₂O is replaced by La(NO₃)₃·6H₂O.

The obtained composite carrier is determined to have a specific surface area of 177 m²/g, a total pore volume of 0.38 cm³/g, and an average pore diameter of 10 nm.

### Preparation Example 5: Preparation of Catalyst D1

The procedure of Preparation Example 1 is repeated, with the only difference that diatomite is replaced by a commercial activated alumina carrier (JL-C-02 activated alumina, Jiulong Chemical). The activated alumina carrier contains no modifier. The activated alumina carrier is determined to have a specific surface area of 170 m²/g, a total pore volume of 0.6 cm³/g, and an average pore diameter of 12 nm.

### Preparation Example 6: Preparation of Catalyst D2

The procedure of Preparation Example 1 is repeated, with the only difference that diatomite is replaced by commercial silica (Q-15 series SiO₂ carrier, Fuji, Japan). The silica carrier contains no modifier. The silica carrier is determined to have a specific surface area of 200 m²/g, a total pore volume of 1.0 cm³/g, and an average pore diameter of 15 nm.

### Preparation Example 7: Preparation of Catalyst D3

The same preparation procedure as in Preparation Example 1 is adopted, with the only difference that the molar ratio of Zr to Ce in the solution is adjusted to 1:0.3, so that a Zr₁Ce_{0.3} modifier is prepared, and catalyst D3: Ni₁₀-Zr₁Ce_{0.3}P is finally obtained.

### Example 1

The process of the present disclosure is carried out in a fixed-bed reactor as shown in FIG. 1. 100 kg of Catalyst 1 prepared in Preparation Example 1 with a bulk density of 990±50 kg/m³ is weighed and loaded into the fixed-bed reactor. Commercially available dimethyl maleate (DMM) raw material (Hengli New Materials Co., Ltd.) is used. The feed composition and feed conditions are listed in Table 1. The feed rate of DMM is 50 kg/h, the molar ratio of DMM raw material to recycled hydrogen is 1:300, the reaction pressure is 3.0 MPaG, the reaction temperature is 190 °C, and the standard gas hourly space velocity of the reactor is about 23300 h⁻¹. The DMM feed is heated to 190 °C by a heater and then instantaneously vaporized after entering a gas-liquid separator. A small amount of un-vaporized heavy component impurities are separated and discharged. The vaporized mixture of DMM and hydrogen is fed into the reactor from the bottom of the reactor. The bed temperature difference of the reactor is monitored through temperature measuring points of the reactor. The reactor bed temperature difference Δt of the fixed-bed reactor is controlled to ≤ 0.1 °C, and the reactor bed pressure drop ΔP of the fixed-bed reactor is ≤ 0.1 MPa.

**Table 1.**

| Component | | Feed conditions | Content (wt%) |
|---|---|---|---|
| Name | English abbreviation | | |
| Maleic anhydride | MAH | DMM feed rate = 50 kg/h | 0.15 |
| Dimethyl maleate | DMM | | 98.06 |
| Dimethyl succinate | DMS | | Trace |
| γ-Butyrolactone | GBL | Feed pressure = 3.2 MPaG | Trace |
| 1,4-Butanediol | BDO | Temperature = 50 °C | Trace |
| Methanol | MET | | 1.78 |
| Heavy components | HEAVIES | | ≤0.01 |

The reactants are withdrawn from the top of the reactor. After heat recovery, the gaseous reactants are condensed into a liquid phase to obtain a crude DMS product, which is subjected to chromatographic analysis. The conversion rate is calculated based on the consumption of DMM, and the selectivity is calculated based on the DMS content in the reaction solution.

The crude DMS reaction solution is fed into a rectification unit for further purification and separation. The first rectification is used for the preliminary separation of crude DMS to remove light components such as methanol and dimethyl ether contained in the crude DMS, and the light components are distilled off from the top of the column. The operating pressure is 20 kPaA and the operating temperature is 165 °C.

The second rectification column is a product column for the final refining of the light-component-removed DMS. The operating pressure is 3 kPaA and the temperature is 172 °C. Industrial-grade DMS is withdrawn from the top of the column, polymerization-grade DMS is withdrawn from the side stream, and heavy components are discharged from the bottom. The purity of the polymerization-grade DMS is 99.93%.

The third rectification column is a light-component column for the recovery of DMS from the light components. Light components are obtained at the top and crude DMS at the bottom. The operating pressure is 10 kPaA and the operating temperature is 168 °C.

### Example 2

The procedure of Example 1 is repeated, with the only difference that Catalyst 1 is replaced by Catalyst 2 prepared in Preparation Example 2.

### Example 3

The procedure of Example 1 is repeated, with the only difference that Catalyst 1 is replaced by Catalyst 3 prepared in Preparation Example 3.

### Example 4

The procedure of Example 1 is repeated, with the only difference that Catalyst 1 is replaced by Catalyst 4 prepared in Preparation Example 4.

### Comparative Example 1

The procedure of Example 1 is repeated, with the only difference that Catalyst 1 is replaced by Catalyst D1 prepared in Preparation Example 5.

### Comparative Example 2

The procedure of Example 1 is repeated, with the only difference that Catalyst 1 is replaced by Catalyst D2 prepared in Preparation Example 6.

### Comparative Example 3

The procedure of Example 1 is repeated, with the only difference that Catalyst 1 is replaced by Catalyst D3 prepared in Preparation Example 7.

### Test Example 1

The crude DMS product obtained in the fixed-bed reactor is directly subjected to component analysis. The contents of each component in the crude DMS and the catalytic performance are shown in Table 2.
1. The DMM conversion and DMS purity are determined using an Agilent 7890 chromatograph. The chromatographic conditions are set as follows:
   Column: CP-SIL8CB, 50 × 0.32 × 1.2;
   Detector temperature: 300 °C;
   Inlet temperature: 250 °C;
   Split ratio: 25:1;
   Column flow rate: 2.4 mL/min.
2. The DMM conversion is calculated according to the following equation: ${X}_{A} = \frac{{A}_{1} - {A}_{2}}{{A}_{1}} \times 100 %$
   X_{A}: conversion based on reactant A;
   A₁: initial mass of reactant A;
   A₂: residual mass of reactant A after reaction;
   reactant A is DMM.
3. The catalyst selectivity based on DMM is calculated according to the following equation: $S = \frac{B}{{A}_{1} - {A}_{2}} \times 100 %$
   S: selectivity;
   B: mass of target product B;
   A₁: initial mass of reactant A;
   A₂: residual mass of reactant A after reaction;
   target product B is DMS, and reactant A is DMM.

**Table 2.**

| No. | Crude DMS after hydrogenation (wt%) | | | | | | | Catalyst performance (%) | |
|---|---|---|---|---|---|---|---|---|---|
| | MAH | DMM | DMS | GBL | BDO | Metha nol | Others | Conversion | Selectivi ty |
| Ex. 1 | 0.15 | 0.03 | 98.02 | 0.0005 | 0.0038 | 1.79 | 0.008 | 99.97 | 99.98 |
| Ex. 2 | 0.14 | 0.07 | 97.92 | 0.0026 | 0.0068 | 1.86 | 0.01 | 99.93 | 99.93 |
| Ex. 3 | 0.15 | 0.06 | 97.92 | 0.0015 | 0.0049 | 1.86 | 0.016 | 99.94 | 99.93 |
| Ex. 4 | 0.14 | 0.05 | 97.88 | 0.0005 | 0.0083 | 1.92 | 0.006 | 99.95 | 99.86 |
| Comp. Ex. 1 | 0.12 | 12.86 | 76.73 | 5.12 | 0.0148 | 5.16 | 0.023 | 86.89 | 90.05 |
| Comp. Ex. 2 | 0.13 | 14.22 | 73.43 | 6.32 | 0.0196 | 5.88 | 0.018 | 85.50 | 87.58 |
| Comp. Ex. 3 | 0.14 | 9.87 | 88.01 | 0.0032 | 0.0098 | 1.95 | 0.016 | 89.96 | 99.80 |

### Test Example 2

The refined DMS product obtained by three-stage rectification is subjected to component and color number analysis.

The content of each component in the product is determined using the same method as that in Test Example 1.

The color number and the content of each component of the DMS product obtained after three-stage rectification are shown in Table 3.

The color number is measured in accordance with GB 3143-1982 using a Lovibond Tintometer PFX190.

**Table 3.**

| Component | Unit | Ex. 1 | Ex. 2 | Ex. 3 | Ex. 4 | Comp. Ex. 1 | Comp. Ex. 2 |
|---|---|---|---|---|---|---|---|
| Color number | APHA | 4 | 5 | 3 | 4 | 13 | 16 |
| DMS | wt% | 99.95 | 99.91 | 99.93 | 99.9 | 90.30 | 89.60 |
| GBL | wt% | Trace | Trace | Trace | Trace | 1.34 | 1.68 |
| BDO | wt% | Trace | Trace | Trace | Trace | 0.12 | 0.10 |
| DMM | wt% | 0.03 | 0.06 | 0.02 | 0.05 | 0.01 | 0.02 |
| Others | wt% | 0.02 | 0.03 | 0.05 | 0.05 | 8.23 | 8.60 |

| | | | | | | | |
|---|---|---|---|---|---|---|---|
| "Trace" means a very small amount with almost no detectable peak, and the content is generally less than 1 ppm. | | | | | | | |

It can be seen from the above analysis results of the hydrogenation reaction (Table 1) that the catalysts of the present disclosure all exhibit excellent hydrogenation performance, and both the conversion and the selectivity are superior to those of other catalysts. The side reaction to form GBL is well suppressed, which provides conditions for obtaining high-purity DMS by rectification. Furthermore, the use of the fixed-bed reactor of the present disclosure allows a significant increase in the cold hydrogen circulation rate without affecting the pressure drop of the reactor, thereby eliminating hot spots in the catalyst bed and effectively inhibiting the formation of various by-products. As a result, the purity of the polymerization-grade DMS obtained after rectification is all ≥ 99.9%.

### Use Example

The DMS product prepared in Example 1, commercially available succinic acid (Shandong Feiyang Chemical Co., Ltd., meeting the requirements of first-grade product specified in T/HNPCIA 13-2019), commercially available succinic anhydride (Hebi Coal Chemical Co., Ltd., Henan Energy Chemical Group Co., Ltd., meeting the requirements of first-grade product specified in GB 34686-2017), the DMS product prepared in Comparative Example 1, and the DMS product prepared in Comparative Example 2 are used as polymerization raw materials in a 200 L batch PBS polymerization test. The specifications of the commercially available succinic acid and commercially available succinic anhydride are shown in Tables 4 and 5.

**Table 4: Specifications of Commercially available Succinic Acid**

| Item | Unit | Analysis Result |
|---|---|---|
| Content | wt% | 99.73 |
| Moisture | w% | 0.28 |
| Melting point | °C | 187.9 |
| Iron content | wt% | 0.0008 |
| Chloride (calculated as Cl⁻) | wt% | 0.0018 |
| Sulfate (calculated as SO₄) | wt% | 0.006 |
| Heavy metals (calculated as Pb) | wt% | 0.0005 |
| Easily oxidizable substances | ml/g | 0.2 |
| Ash content | wt% | 0.0091 |

**Table 5: Specifications of Commercially available Succinic Anhydride**

| Item | Unit | Analysis Result |
|---|---|---|
| Content | wt% | 99.58 |
| Melting point | °C | 116.98 |
| Free succinic acid | wt% | 0.8 |
| Unsaturated compounds (calculated as maleic anhydride) | wt% | 0.0081 |
| Chloride (calculated as Cl⁻) | wt% | 0.0018 |
| Sulfate (calculated as SO₄) | wt% | 0.0020 |
| Heavy metals (calculated as Pb) | wt% | Trace |
| Residue on ignition | wt% | 0.004 |

Charging: The above polymerization raw materials and BDO are charged into the reactor at a molar ratio of 1:1.2 and mixed under stirring. If the dissolution is insufficient, heating may be performed at 140 °C for 1 hour. The mixed slurry is then transferred into the reactor for reaction.

The reaction pressure is 10 kPaG. The reaction temperature is 160±2 °C. A sample of the reaction solution in the reactor is taken every 1 hour, and the DMS content in the reaction solution is analyzed. After about 6 hours, the reaction is terminated when the DMS content decreases to below 500 ppm and remains stable. The reaction solution is transferred into a pre-polycondensation reactor for pre-polycondensation.

Pre-polycondensation: Tetraisopropyl titanate (TtiPO, CAS: 546-68-9) is used as the catalyst, at a molar ratio of catalyst to polymerization raw materials of 1:400. The reaction system is heated to 210 °C and maintained at 1 kPaA for 3 h to obtain a pre-polycondensate. All the reaction solution in the pre-polycondensation reactor is fed into the final polycondensation reactor.

Final polycondensation: The reaction pressure is 65±2 PaA. The reaction temperature is 225±5 °C. The stirring speed is 210 rpm. The final polycondensation is completed when the power of the stirrer no longer increases and slightly decreases, with a reaction time of about 3 h. The stirring speed is properly reduced during the reaction to prevent excessive shear heat.

Pelletizing: The polycondensation product obtained in the final polycondensation step is dried and pelletized to obtain the finished PBS product. Nitrogen is introduced at the end point, and the product is discharged and pelletized. The whole pelletizing process is performed under clean conditions to avoid external contamination.

### Use Test Example

The properties of PBS prepared in the Use Example are tested using the following methods. The test results are shown in Table 4.

Terminal carboxyl group content refers to the content of carboxyl functional groups near the two ends of PBS molecules, and is mainly used to characterize information such as average molecular weight. The level of terminal carboxyl group content directly affects the thermal stability, degradation rate, and color number of the product. Terminal carboxyl group content is determined by titration-photometry. A sample is dissolved under reflux in a mixed solvent of o-cresol and CHCl₃. After cooling, bromophenol blue is used as an indicator, and the sample is titrated with a standard sodium hydroxide-ethanol titrant. The terminal carboxyl group content of PBS is calculated based on the consumption of the standard titrant. The formula is as follows: $X = \frac{\left(V-{V}_{0}\right) c \times {10}^{3}}{m}$ Wherein:
*V* denotes the volume of sodium hydroxide-ethanol standard titrant consumed by the sample, mL;
*V₀* denotes the volume of sodium hydroxide-ethanol standard titrant consumed in the blank test, mL;
*c* denotes the concentration of sodium hydroxide-ethanol standard titrant, mol/L;
*m* denotes the weighed mass of the sample, g.

Melt flow index is measured according to ASTM-D1238 using a Ray-Ran MFR300.

Oxidation resistance is characterized by oxidation induction time and oxidation induction temperature, according to ASTM D3895-14, using a Mettler Toledo TGA/DSC 3⁺ differential scanning calorimeter. To eliminate the thermal history of the sample, the sample is first heated to 150 °C at 20 °C/min and kept isothermal for 5 min, then cooled to 25 °C at a rate of 20 °C/min before the test is performed. A sample with a mass of 5.5 ± 0.5 mg is heated to 220 °C under a nitrogen atmosphere. After being held isothermally at 220 °C for 5 min, the atmosphere is switched from nitrogen to oxygen (both gases at a flow rate of 50 mL/min), and the temperature is maintained for 120 min or until a peak appears in the DSC curve. An uncovered aluminum crucible is used. All samples are measured in duplicate.

It can be seen from Table 6 that PBS plastics prepared using DMS from Example 1 as raw material exhibit better performance than PBS prepared using raw materials from Comparative Examples 1-2, commercially available succinic acid, and commercially available succinic anhydride.

**Table 6.**

| Polymerization raw material | Terminal carboxyl group content (mol/t) | Melt flow index (g/min) | Oxidation induction time (min, isothermal OIT) | Oxidation induction temperature (°C, dynamic OIT) |
|---|---|---|---|---|
| DMS-Example 1 | 45.8 | 6.2 | 116 | 299 |
| Succinic anhydride | 31.3 | 5.6 | 58 | 246 |
| Succinic acid | 21.6 | 4.4 | 22 | 216 |
| DMS-Comparative Example 1 | 9.2 | 2.4 | 16 | 196 |
| DMS-Comparative Example 2 | 5.1 | 1.4 | 12 | 182 |

In summary, the catalyst of the present disclosure and the process for hydrogenating dimethyl maleate to produce dimethyl succinate provided by the present disclosure effectively improve the selectivity to DMS and enhance the purity of the DMS product. Further, by using the fixed-bed reactor of the present disclosure to control the bed temperature difference, energy consumption and production cost are reduced, thereby improving the economic benefit of the production of the degradable plastic PBS.

## Claims

1. A catalyst, wherein the catalyst comprises a composite carrier and an active metal Ni loaded on the composite carrier; the composite carrier is formed by compounding a modifier and a natural mineral, wherein the modifier comprises zirconium and a rare earth metal; the natural mineral is one or more selected from diatomite, sepiolite, halloysite, attapulgite, vermiculite and molybdenite; the rare earth metal is one or more selected from yttrium, lanthanum, cerium, neodymium, samarium, europium, gadolinium and ytterbium; a molar ratio of zirconium to the rare earth metal in the modifier is 1:(0.001-0.5); a content of zirconium element is 0.01-30wt% based on the weight of the natural mineral; a content of the active metal Ni in the catalyst is 1-40wt%.

2. The catalyst according to claim 1, wherein the catalyst has one or more of the following features:
the modifier consists of zirconium and the rare earth metal;
the molar ratio of zirconium to the rare earth metal in the modifier is 1:(0.01-0.1);
the content of zirconium element is 0.5-20wt% based on the weight of the natural mineral;
the content of the active metal Ni in the catalyst is 5-25wt%;
the natural mineral is one or more selected from diatomite and sepiolite;
the rare earth metal is one or more selected from yttrium, lanthanum and cerium;
a specific surface area of the composite carrier is 150-450 m²/g;
a total pore volume of the composite carrier is 0.15-1.5 cm³/g;
an average pore diameter of the composite carrier is 5-30 nm.

3. The catalyst according to claim 1, wherein the catalyst is used for catalyzing gas-phase hydrogenation of dimethyl maleate to produce dimethyl succinate.

4. The catalyst according to claim 1, wherein the modifier is prepared from an aqueous solution containing a zirconium salt and a rare earth metal salt by a high-pressure hydrothermal method.

5. The catalyst according to claim 1, wherein the catalyst is represented by a general formula Niₓ-Zr_{y}R_{z}P, R represents a rare earth metal, P represents a natural mineral, x represents that a mass fraction of Ni element in the catalyst is x%, and y and z represent that a molar ratio of Zr to R is y:z;
the catalyst is Niₓ-Zr_{y}Ce_{z}P, wherein P is sepiolite; or the catalyst is Niₓ-Zr_{y}Y_{z}P, wherein P is diatomite; or the catalyst is Niₓ-Zr_{y}La_{z}P, wherein P is diatomite.

6. A method for preparing the catalyst according to any one of claims 1-5, wherein the method comprises steps of:
(1) preparing a modifier from an aqueous solution containing a zirconium salt and a rare earth metal salt by a high-pressure hydrothermal method;
(2) forming a precipitate from the modifier and the natural mineral in a dispersant, filtering and collecting the precipitate, drying and calcining the precipitate to obtain a composite carrier;
(3) impregnating the composite carrier in an aqueous solution containing a nickel salt, filtering off the solution, drying and calcining to obtain a catalyst precursor;
(4) subjecting the catalyst precursor to a reduction reaction in a reducing gas to obtain the catalyst.

7. The method according to claim 6, wherein step (1) has one or more of the following features:
the zirconium salt is one or more selected from zirconium nitrate, zirconium chloride and zirconium alkoxide;
the rare earth metal salt is one or more selected from Gd(NO₃)₃·6H₂O, Yb(NO₃)₃·5H₂O, Y(NO₃)₃·4H₂O, La(NO₃)₃·6H₂O, Ce(NO₃)₃·6H₂O, Nd(NO₃)₃·6H₂O, Sₘ(NO₃)₃·6H₂O, Eu(NO₃)₃·5H₂O and chlorides of the rare earth metals;
a concentration of the zirconium salt in the aqueous solution is 2-2.5 mol/L;
the molar ratio of zirconium to the rare earth metal in the aqueous solution is 1:(0.001-0.5);
a reaction pressure of the high-pressure hydrothermal method is 1.0-3.0 MPa;
a reaction temperature of the high-pressure hydrothermal method is 110-150 °C;
a reaction time of the high-pressure hydrothermal method is 3-24 h.

8. The method according to claim 6, wherein step (2) has one or more of the following features:
the modifier is added in an amount such that a content of zirconium element in the modifier is 0.01-30wt% based on the weight of the natural mineral;
the dispersant is one or more selected from methanol, ethanol and water;
100-500 g of the natural mineral is added per liter of the dispersant;
the conditions for forming the precipitate are: dispersing the modifier and the natural mineral under stirring with heating, followed by standing under heating until a precipitate is formed; preferably, the stirring and the standing are each independently carried out at 50-90 °C;
a drying temperature is 100-150 °C;
a drying time is 2-48 h;
a calcination temperature is 150-800 °C;
a calcination time is 0.5-12 h;
prior to performing step (2), the natural mineral is subjected to heat treatment and/or chemical treatment; preferably, a temperature of the heat treatment is 300-800 °C; preferably, the chemical treatment is soaking the natural mineral in an acid solution.

9. The method according to claim 6, wherein step (3) has one or more of the following features:
the nickel salt is one or more selected from nickel nitrate, nickel chloride and nickel sulfate;
the aqueous solution containing a nickel salt further comprises an organic acid;
a concentration of the nickel salt in the aqueous solution containing a nickel salt is 0.01-2.0 g/mL;
an impregnation time is 20-240 min;
a drying temperature is 80-120 °C;
a drying time is 2-12 h;
a calcination temperature is 150-550 °C;
a calcination time is 0.5-50 h.

10. The method according to claim 6, wherein step (4) has one or more of the following features:
the reducing gas comprises hydrogen and a protective gas, wherein the protective gas is one or more selected from nitrogen, helium and argon;
a temperature of the reduction reaction is 130-650 °C;
a system for the reduction reaction is heated to a set temperature at a heating rate of not more than 10 °C/h;
after the temperature of the system for the reduction reaction reaches the set value, the system is maintained for 4-48 h.

11. A method for preparing dimethyl succinate, wherein the method comprises step (a): subjecting dimethyl maleate to hydrogenation reaction with hydrogen under the catalysis of a catalyst to obtain a crude dimethyl succinate, wherein the catalyst is the catalyst according to any one of claims 1-5 or the catalyst prepared by the method according to any one of claims 6-10.

12. The method according to claim 11, wherein the method further comprises step (b): rectifying the crude dimethyl succinate to obtain dimethyl succinate;
preferably, in step (b), the rectification is three-stage rectification; the three-stage rectification comprises a first-stage rectification, an operating pressure of the first-stage rectification is 5-50 kPaA, and an operating temperature of the first-stage rectification is 130-180 °C; the three-stage rectification comprises a second-stage rectification, an operating pressure of the second-stage rectification is 0.1-10 kPaA, and an operating temperature of the second-stage rectification is 130-200 °C; the three-stage rectification comprises a third-stage rectification, an operating pressure of the third-stage rectification is 1-30 kPaA, and an operating temperature of the third-stage rectification is 110-170 °C.

13. The method according to claim 11, wherein step (a) has one or more of the following features:
a molar ratio of dimethyl maleate to hydrogen is 1:(100-500);
a reaction pressure is 1.0-10.0 MPaG;
a reaction temperature is 110-230 °C;
a gas hourly space velocity of a reactor is 20000-30000 h⁻¹;
in step (a), a conversion rate of dimethyl maleate is ≥99.9%;
in step (a), a selectivity to dimethyl succinate is ≥99%;
in step (a), a content of γ-butyrolactone in the crude dimethyl succinate is ≤5wt%.

14. A method for preparing polybutylene succinate, wherein the method comprises steps of:
(A) providing the catalyst according to any one of claims 1-5, or providing the catalyst prepared by the method according to any one of claims 6-10;
(B) subjecting dimethyl maleate to a hydrogenation reaction with hydrogen under catalysis of the catalyst to prepare dimethyl succinate;
(C) subjecting the dimethyl succinate prepared in step (B) to polymerization reaction with 1,4-butanediol to obtain polybutylene succinate.

15. The method according to claim 14, wherein step (C) comprises steps of:
(C1) subjecting dimethyl succinate and 1,4-butanediol to transesterification;
(C2) subjecting a reaction solution obtained after completion of the reaction in step (C1) to pre-polycondensation;
(C3) subjecting a reaction solution obtained after completion of the reaction in step (C2) to final polycondensation;
a reaction pressure of the transesterification is 5-20 kPaG;
a reaction temperature of the transesterification is 140-200 °C;
a reaction time of the transesterification is 3-10 h;
the transesterification is terminated when a content of dimethyl succinate in the reaction system is detected to be below 500 ppm;
the pre-polycondensation is carried out under catalysis of a polymerization catalyst; preferably, the polymerization catalyst is a Ti-containing catalyst;
a molar ratio of the polymerization catalyst to dimethyl succinate fed in step (C1) is 1:(300-500);
a reaction temperature of the pre-polycondensation is 180-250 °C;
a reaction pressure of the pre-polycondensation is 0.5-2 kPaA;
a reaction time of the pre-polycondensation is 2-5 h;
a reaction pressure of the final polycondensation is 50-80 PaA;
a reaction temperature of the final polycondensation is 200-250 °C;
a reaction time of the final polycondensation is 1-5 h;
the final polycondensation is completed when a viscosity of the reaction system no longer increases.
